# EUROPEAN PATENT APPLICATION

(11) **EP 0 839 550 A1**
(43) Date of publication of application: **06.05.1998**
(21) Application number: 97308638.2
(22) Date of filing: 29.10.1997
(51) Int. Cl.: A61M 25/10

(54) **Retractable perfusion-occlusion bypass catheter**

(30) Priority: 30.10.1996 CA 2189190
(71) Applicant: Ottawa Heart Institute Research Corporation, Ottawa, Ontario, K1Y 4A8 (CA)
(72) Inventor: Mussivand, Tofy, Navan, Ontario, K4B 1H9 (CA); Gill, Inderjit, Nepean, Ontario K2G 5Y5 (CA)
(74) Representative: Adams, Nicola

(57) **Abstract**

A blood stopping catheter comprises first and second elongate tubular members and first and second inflatable balloons disposed in a spaced relation along the first elongate tubular member. The second elongate tubular member communicates with the first and second inflatable balloons for inflation thereof so that when the catheter is inserted in an artery through an incision the first and second balloons are positioned on each side of the incision, and inflating the first and second balloons stops the blood flow from the incision while allowing blood flow through the first elongate tubular member.

## Description

This invention relates to devices for use in stopping blood flow and in particularly concerned with use in the coronary artery.

### BACKGROUND OF THE INVENTION

Coronary artery bypass surgery has become routine in major hospitals through the use of complex procedures supported by high technology equipment. In order to perform this procedure, a circulatory support system apparatus such as the cardiopulmonary bypass (CPB) replaces the lungs and heart of the patient undergoing surgery when the heart is stopped to perform surgery. The use of this apparatus involves additional surgical and technical manouvers and leads to increased complications for the patient. Thus there is a need for a simpler approach, by using a simple device.

### The Problem

Heart disease that causes the reduction of blood supply to the heart muscle is called coronary artery disease. Coronary artery disease (CAD) can be manifested by chest pain (angina) or heart attack (myocardial infarction) causing death of the heart muscle. The coronary arteries become restricted (narrowed) by the build up of fatty deposits (atherosclerosis) from the accumulation of excess fat and cholesterol in the blood, causing plaque (masses of deposition) build up. This plaque can cause the thickening of the arterial wall. The cross sectioned area of the arteries become narrowed and restricts blood flow. Restricting blood flow causes an increase in velocity causing transition flow forms, turbulence stasis and other adverse blood flow patterns.

Turbulence and other adverse flow can further increase endothelial damage (cell lining). This attracts platelets and blood cells, thus creating the beginning of thrombosis (blood clotting). Eventually, this can completely block the arteries.

Atherosclerosis will occur anywhere along the coronary arteries. Generally, plaque builds at locations where blood forces (shear forces) are dominant (i.e., bifurcation - branches/junctions between main arteries)

### CHD (Coronary Heart Disease)

The restriction (narrowing) of the arterial lumen supplying the heart muscle with blood can cause coronary heart disease. A major manifestation of coronary heart disease can be:
1. **Heart attack.** Death of an area of heart muscle caused by deprivation of blood supply. Technically, this is called myocardial infarction.
   This can occur suddenly and is more severe and not always initiated by physical and/or mental exertion. Generally, this is not relieved by resting (unlike angina). There may be sweating, weakness and sometimes loss of consciousness. When a heart attack completely stops the heart, it is referred to as cardiac arrest which usually leads to death.
   A heart attack is caused by blockage of the coronary artery. When blood flow is reduced or completely stopped, the heart muscle dies. The severity of this death depends on the amount of muscle affected. When a heart attack occurs, the damaged muscle releases certain enzymes into the blood stream. Measuring enzyme activity can help in the determination of the damage to the heart muscle. These enzymes from heart muscle fibres enter into the capillaries and travel through the coronary veins into general circulation. A number of different enzymes are released from the damaged heart muscle.
2. **Angina.** Heavy, gripping chest pain, generally associated with physical exercise.
   Angina is chest pain that comes with exertion and is an indication of low blood flow to the heart muscle. It is typically a gripping pressure-like pain under the breast bone (sometimes radiates into other parts of the body, for instance neck, jaw, arms). The pain is generally relieved with rest.

### Causes of Angina

When the blood supply is insufficient, the heart's supply of oxygen and glucose is diminished. The heart tries to create its own energy by going through a different chemical process than when there is sufficient oxygen. This produces wastes that can not be adequately removed with low blood flow. These substances cause pain to be manifested.

### SUMMARY OF THE INVENTION

This invention relates to the device for use in local occlusion (stopping) and delivery of cardioplegia and/or local myocardial blood flow for use in minimally invasive coronary artery surgery. These devices will allow the surgeons to perform coronary bypass through a small incision in the left chest by using the device. This surgery can be done on a beating heart, thus eliminating the need for cardiopulmonary bypass, eliminating the need or cardiopulmonary bypass, reducing blood trauma, and will substantially reduce complication and cost.

According to the invention, there is provided an inflatable double balloon bypass catheter that can be retracted when deflated. The device can be inserted into the coronary artery and:
1. can occlude locally and perfuse at a distal location while the balloons are inflated. Thus local surgery can be performed in a blood less field while distal perfusion is maintained to prevent ischemia;
2. can allow local delivery of myocardial stunning agents that can cause temporary cessation of motion of local segmental myocardium for very short periods of time while the anastomosis is being performed.

According to one aspect of the present invention there is provided a blood stopping catheter comprising: a first elongate tubular member; first and second inflatable balloons disposed in a spaced relation along the first elongate tubular member; and a second elongate tubular member communicating with said first and second inflatable balloons for inflation thereof.

According to another aspect of the present invention there is provided a use of a blood stopping catheter comprising a first elongate tubular member; first and second inflatable balloons disposed in a spaced relation along the first elongate tubular member; and a second elongate tubular member communicating with said first and second inflatable balloons for inflation thereof so that when the catheter is inserted in an artery through an incision the first and second balloons are positioned on each side of the incision, and inflaming the first and second balloons stops the blood flow from the incision while allowing blood flow through the first elongate tubular member.

### BRIEF DESCRIPTION OF THE DRAWINGS

The present invention will be further understood from the following description with reference to the drawings in which:
Fig. 1 in a flow chart illustrates the atherosclerosis process;
Fig. 2 illustrates known cardiopulmonary bypass apparatus as used with a patient during bypass surgery;
Fig. 3a in a plane view illustrates a blood flow stopper in according with a first embodiment of the present invention;
Fig. 3b illustrates the embodiment of Fig. 3a in an inflated state;
Figs. 4a, b, c, d illustrate insertion of the device of Fig. 3a into a blood vessel;
Fig. 5 illustrates a detailed view of Fig. 4d;
Fig. 6 illustrates a detailed view of Fig. 4d for an alternative embodiment; and
Fig. 7 illustrates a cross-section view of the tubular member and inflation tube of Fig. 5, taken along the line A-A in Fig. 5.

### DETAILED DESCRIPTION OF PREFERRED EMBODIMENTS

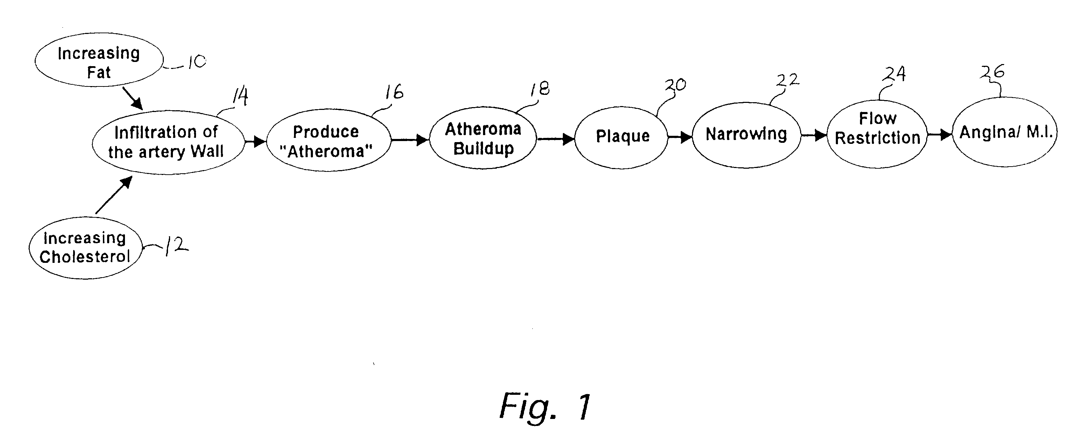

Referring to Fig. 1 there is illustrated in the flow chart the atherosclerosis process. The process begins with increasing fat and cholesterol as represented by blocks 10 and 12 infiltrating the artery wall as represented by block 14 to produce atheroma as represented by block 16. The atheroma builds up over time as represented by block 18 to form plaque causes a narrowing of the artery as represented by block 22 with consequent flow restriction as represented by block 24 leading to conditions such as angina and heart attack. Approximately 80% of the adult population in North America is affected by this condition in varying degrees. Advanced stages of the condition necessitate coronary artery bypass surgery.

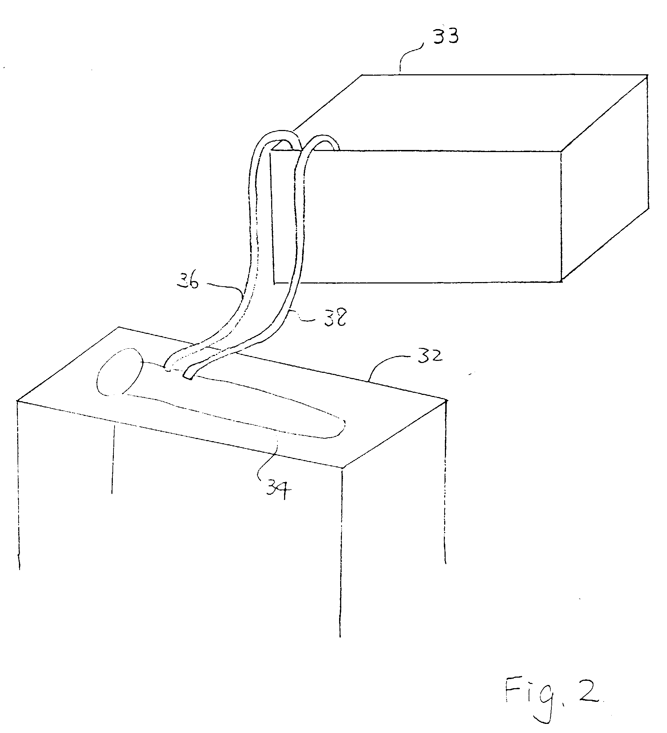

Referring to Fig. 2 there is illustrated a known cardiopulmonary bypass apparatus as used with a patient during bypass surgery. The cardiopulmonary bypass apparatus 30 (not shown) is placed adjacent 18 to the operating table 32 and attached to patient 34 via tubing 36 and 38. During the operation the heart is stopped and the cardiopulmonary bypass apparatus replaces the function of the heart and lungs of the patient. The use of this apparatus is not without risk of complications both during surgery and during the post operative period. Damage to blood cells and platelets filtered and oxygenated in the apparatus can lead to bleeding complications of varying severity. This results in an increased requirement for transfusions to replace the lost blood and blood products which also increases the risk of infection, such as hepatitis transmitted diseases such as HIV and other complications.

In addition, the apparatus itself is expensive and complex limiting its accessibility to larger institutions. With increasing concerns over escalating medical costs, there is a need to develop more cost effective techniques that also eliminates medical complications.

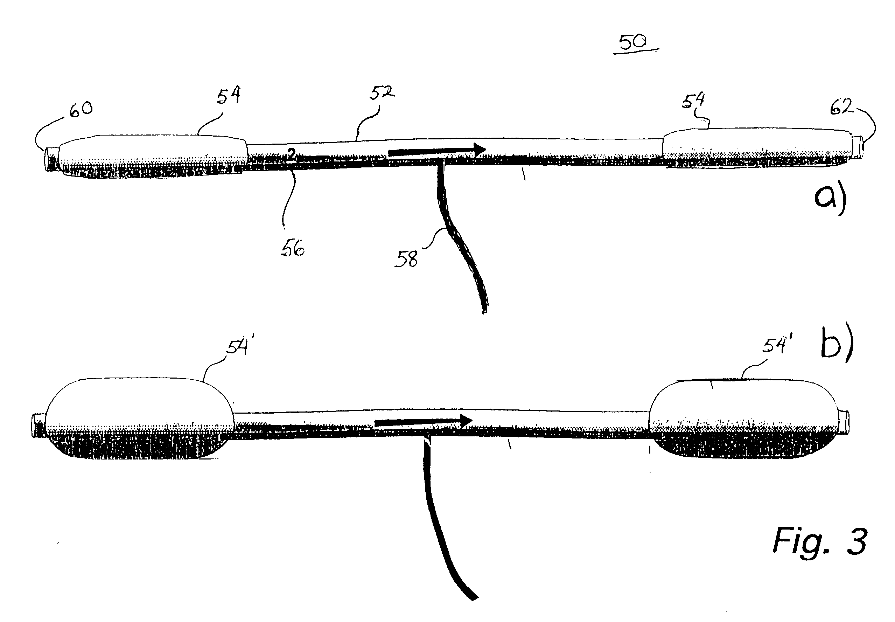

Referring to Fig. 3a, there is illustrated in a plane view a blood flow stopper in accordance with the first embodiment of the present invention. The blood flow stopper 50 includes an elongate tubular member 52 having a pair of balloon members 54 disposed at opposite ends thereof and an inflation tube 56 and a coupling tubing 58. The blood flow stopper includes an inlet 60 and an outlet 62.

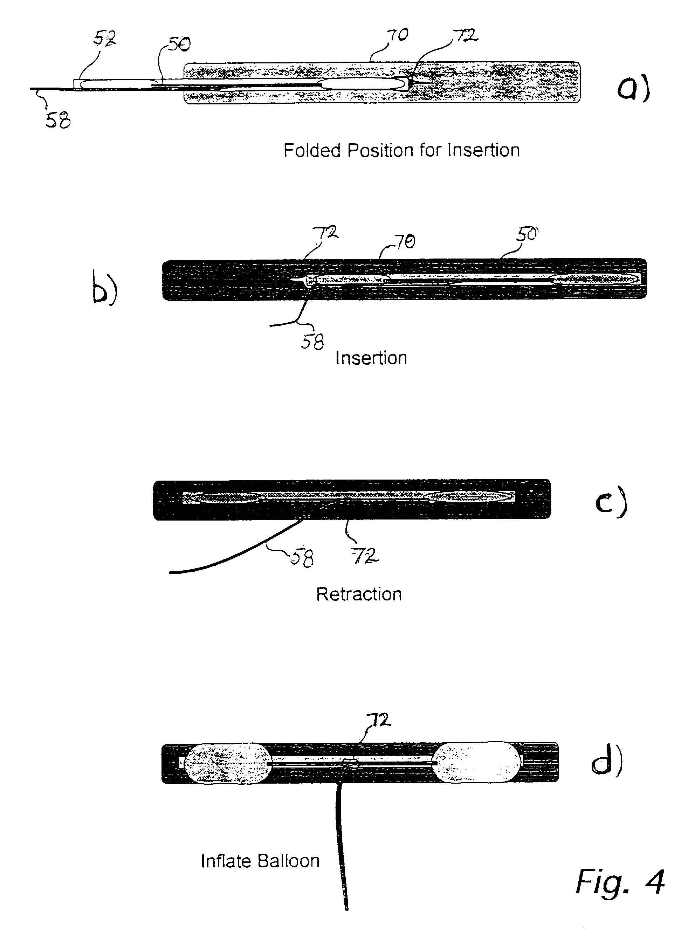

The elongate tubular member 52 is flexible and may be made from flexible polyurethane, Hexyn or Biolon. In a particular embodiment the elongate tubular member 52 has an outside diameter of 1.5 mm, and the balloon members 54 have an outside diameter of 3.5 mm when inflated under a pressure of 150 mn Hg. Referring to Fig. 3b, the balloon members are shown in inflated condition 54'. Referring to Figs. 4a, b, c, and d illustrate insertion of the embodiment of Fig. 3a into a blood vessel 70. As shown in Fig. 4a, and incision 72 is made in the blood vessel 70, the blood stopper 50 and the coupling tubing 58 are in a folded condition for insertion. In Fig. 4b, a cross sectional view of the vessel 70 is shown with the blood flow stopper 50 in an inserted position. In Fig. 4c, the blood stopper has been retracted by pulling upon the coupling tubing 58 until it is in a final position and in Fig. 4d, the inflation tubing is used to inflate balloon members 54 to achieve a blood stopping condition in a section of the vessel 70 there between.

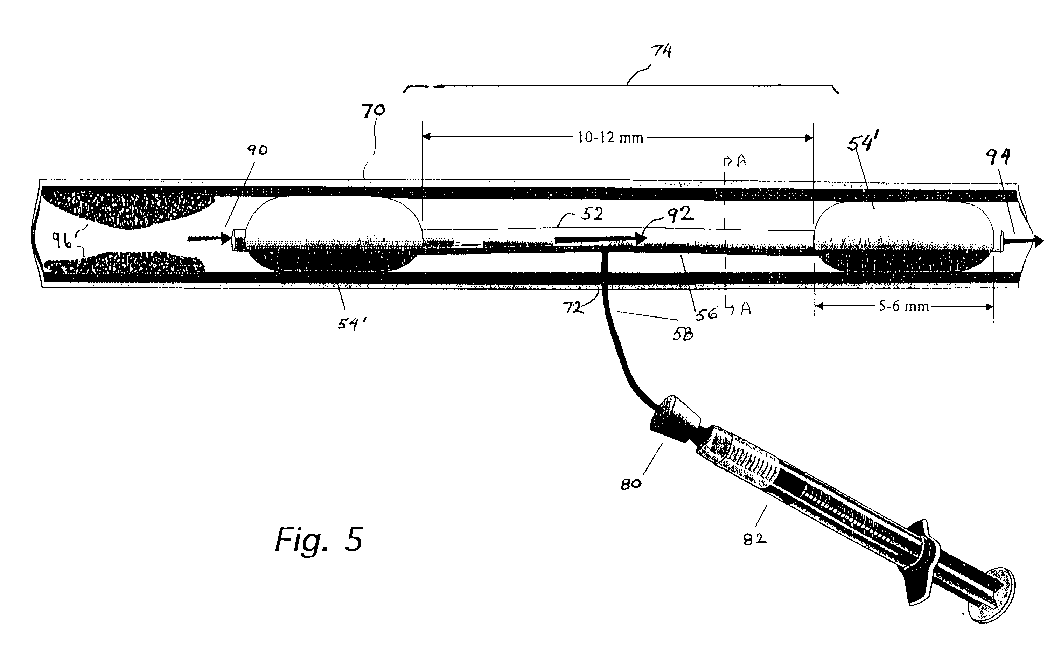

Referring to Fig. 5, there is illustrated a detailed view of Fig. 4d. Fig. 5 illustrates the luer-lock connection of the coupling tubing 58 to a syringe 82 using a syringe stopper 80. Once inflated, the elongate tubing 52 allows blood flow as indicated by arrows 90, 92 and 94, while stopping blood flow from section 74 of the artery. The building up of plaque on the artery wall is represented by 96. Provided the vessel is sufficiently open to allow blood flow, the embodiment of Fig. 5 can be used.

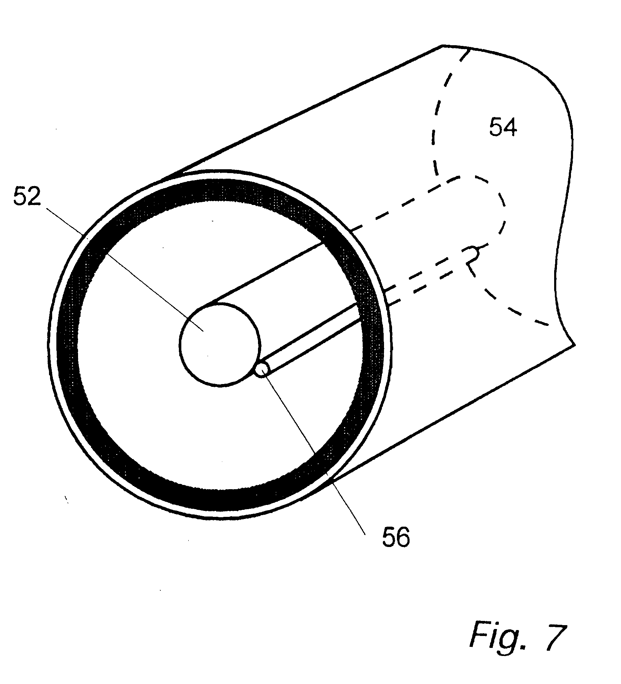

Fig. 7, illustrates the double lumen structure, namely, elongate tubular member 52 for blood flow and the inflation tube 56 for inflation of the balloon members.

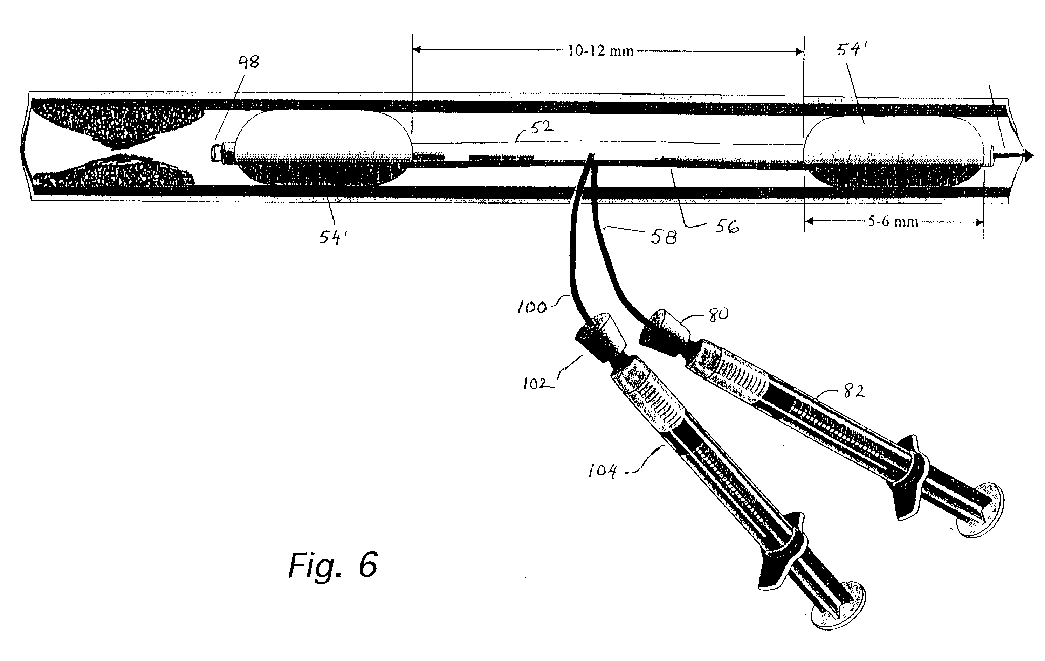

The embodiment of Fig. 6 includes in addition to the components of the embodiment illustrated in Fig. 5 a stopper 98 in the inlet 60 of elongate tube 52. Tubing 100 communicating with tubing 52 and coupled to a second syringe 104 using a second syringe stopper 102. This will allow perfusion of myocardial stunning agents to achieve local cessation of myocardial activity.

### Causes of CHD

CHD is the manifestation of narrowing of the coronary arteries by atherosclerosis. An accumulation of; a) fat, b) cholesterol or c) both, in the blood, can cause the build up of fatty deposits in the lining of blood vessels. When fat and cholesterol accumulate in the blood, they infiltrate the lining of the arteries and slowly build up deposits, referred to as atheroma.

Atheroma enlarges to become a mass of plaque. When plaque and the fibrous cap thickens the arterial wall this narrows the opening (lumen) of the artery, thus restricting blood flow which causes a reduction of oxygen and nutrients to reach the heart muscle. The reduction in cross section increases velocity components and creates adverse flow patterns such as turbulence, stasis and recirculation. Turbulent forces and other adverse flows may erode (damage) the plaque surface. Platelets can accumulate along the roughened surface, forming thrombosis or blood clots. This will further restrict blood flow to the heart muscle and/or can become lodged and create emboli.

### Alternative Treatments to Opening the Coronary Artery

The major methods of treatment are outlined below.

### Drug Treatment.

Medication can be used to dilate the coronary arteries. The dilation of coronary arteries will increase blood flow. Drugs such as nitrates, beta blockers and calcium channel blockers are examples of drugs used for dilating the coronary artery.

### Coronary Artery Surgery.

A narrowed or blocked coronary artery can be treated surgically by:
a. Angioplasty: angio (meaning vessel) and plasty (meaning repair).
b. Bypassing the blockage

### Angioplasty.

Angioplasty is a method used to dilate the coronary artery cross sectional area. This is suitable for patients with single artery restriction or narrowness and is the preferred procedure for some patients unable to withstand surgery (i.e., advanced lung disease and others).

An opening (incision) is made into the leg or arm of the patient. Through this opening, a guide wire, guided by X-ray, is pushed into the brachial (arm) or femoral (leg) artery. The wire is threaded and advanced to the affected areas of the vessel via the aorta. At the narrowed and/or blocked section, the wire is carefully manoeuvred. The catheter is advanced until the tip of the balloon is placed at the obstruction. Liquid or air is forced along the catheter into the balloon, inflating the balloon. This pressure is exerted on the blockage for up to 60 seconds and then released. The pressure can be as high as 8 times atmospheric pressure (760 mm Hg). The radially distributed pressure will push the blockage back, opening the blood flow path. The procedure can be continued until the cross section is widened. This pressure squeezes the plaque against the artery walls and the blood begins to flow (higher discharge). Narrowing of the artery will recur in more than 30 of these patients within 3 months.

### Coronary Artery Bypass.

This is a surgical technique by which the narrowed or blocked vessels are bypassed and is mainly applied to critically narrowed or blocked coronary arteries in patients who are not probable candidates for angioplasty or patients with multiple blockages. Generally, the operation involves the use of the patient's arteries or veins as bypassing tubes. So far, scientists have been unable to produce an artificial vascular graft that can remain patent over the long term. Generally, the left internal mammary artery (LIMA) or saphenous vein are taken (harvested) to be used as bypass tubes. These vessels are used to bypass the blocked section of the diseased artery. With the surgical method, a chest incision (sternotomy) is performed. This surgery requires cardiopulmonary bypass (heart/lung machine) to temporarily take over blood circulation.

The procedure begins with the preparation of the patient. After general anaesthesia, the chest is opened by making an incision along the breast bone and cutting the breast bone. The pericardium (sac surrounding the heart) is opened and the heart exposed. At the same time, an incision is made in the leg to remove a portion of the vessel for the bypass and/or a mammary artery is harvested. The patient is heparinized and the ascending aorta and the right atrium cannulated and the patient put on cardiopulmonary bypass. The cardiopulmonary bypass will take the blood from the patient where it is filtered and oxygenated through an oxygenator and heart/lung machine. The aorta is clamped to prevent blood from reaching the heart and a solution injected to paralyze (stop) the heart. While the heart is stopped, circulation is maintained through the heart/lung machine. The surgeon grafts (sutures) the vein or artery between the aorta and downstream of the narrowed or blocked artery, thus bypassing the obstruction. If more than one artery is obstructed, a similar arrangement will be made for another bypass. Once the surgery and anastomosis (connection) is complete, the heart/lung machine is disconnected and the chest closed.

### Coronary Artery Bypass Associated Risks.

The splitting of the sternum, aortic clamping and cardiopulmonary bypass can have an adverse impact upon the patient (mechanical trauma to the blood vessel, impaired hemostasis, diminished oxygen delivery). Bleeding complications, sternal wound infection, increased risk of atherosclerotic complications causing stroke, renal failure, etc. and total body inflammatory response due to alteration of the body's immune and complement system from the extro-corporeal circuit are examples of some of the problems that arise.

Attempts have been made by many investigators to invent a procedure and/or technology that can reduce the amount of surgery and trauma. Minimally Invasive Direct Coronary Bypass (MIDCB) is an innovative approach. Unlike conventional surgical bypass, the MIDCB procedure does not require the use of a heart/lung machine, splitting of the sternum (breast bone) or aortic clamping. The patients normal hospital stay is 7 - 8 days.

### Minimally Invasive Direct Coronary Bypass (MIDCB) Procedure.

The patient is prepped and prepared as for normal aoro-coronary bypass and is positioned flat on the operating table. Anaesthesia is induced using a regular endo-tracheal tube. After the draping, a 10 cm incision is made below the left nipple and after incising the muscle, a portion of the fourth costal cartilage is excised. The internal mammary artery is then harvested using electrocautery distally up to the fifth rib, and proximally up to the second intercostal space. The patient is then partially heparinized and the internal thoracic artery is taken down, the pericardium is opened and the left anterior descending artery is assessed and snared proximal and distal to where the anastomosis is to be constructed. The heart is stabilized by the second assistant by straddling the artery on both sides with his fingers. An Adenosine bolus is given (12 mg IV bolus) to stop the heart temporarily for four or five seconds and an arteriotomy is then made and the anastomosis is constructed with 8/0 Prolene on the beating heart. After this the distal snare is released and the flow into the internal thoracic artery is assessed using a transonic flow probe. Protamine is given and the incision closed. The patient is extubated on the table and sent to the recovery room, and discharged form the hospital the next day.

### MIDCAB Risks.

The MIDCAB has some associated risks, the main ones being:
1. Ischemia of the myocardium beyond the distal snare due to lack of blood flow during surgery.
2. Difficulty in performing surgery on the beating heart.

In addition to the above risks, additional precautions can result in added costs incurred with heart/lung machine (CPB) equipment and a perfusionist on stand-by. The above risks and standby costs will be eliminated and/or reduced if:
1. Provide a means to perfuse distal tissue, thus preventing the risk of ischemia and cardiac arrest.
2. Deliver local cardiac muscle stunning with short acting agents causing cessation of contractility at the local area. Such is the object of this invention.

### Retractible Blood Flow Bypass Catheter for Minimally Invasive Direct Coronary Bypass.

Double balloon bypass catheter to allow minimally invasive coronary artery bypass grafting has been developed. This permits blood flow to continue during bypass surgery without obstructing the surgical working field. The device will allow downstream myocardial tissue to be perfused during surgery, avoiding risk of ischemic complications (e.g., serious arrhythmia, fall in blood pressure) and increase the safe time during which the surgeon can perform the anastomosis carefully.

The device can also be used as an intra-coronary catheter for local delivery of cardioplegia or other myocardial stunning agents.

This device catheter will have a 1 mm luminal diameter with a proximal balloon that will be occlusive and a distal balloon that would stop any retrograde flow of blood plus allow antegrade flow of cardioplegia or some other solution to cause temporary stunning of segmental myocardium.

The solutions that could be used could be varied forms of cardioplegia or Adenosine which is a potent but short-acting agent that causes complete cessation of contractility through the area which it perfuses for 5 - 10 seconds or more.

Thus, this catheter will allow complete cardiac standstill in the region which is being operated upon.

The principal advantages of this would be avoiding all the associated complications of extracorporeal circuits and cross-clamping and at the same time would have all the advantages that go with the minimally invasive bypass surgery.

### Uniqueness of Retractive Bypass Catheter.

1. Permits blood flow from upstream to downstream of the narrowed luminal section (which is subjected for surgical bypass).
2. Retractability allowing the enhancement of manoeuvrability and placing of the catheter in the lumen of the coronary artery, yet not being obstructive in the operative field.
3. Dual inflatable balloons at the extension of the catheter for simultaneous occlusion of the artery permitting working space for surgeons.
4. Simultaneous inflation pressure distribution upstream and downstream of the balloon.
5. Gentle balloon occlusion of the desired sections of the artery without damaging the inner surface of the artery. A gentle increase or reduction of pressure within the balloon.
6. Strong balloon material capable of standing high radial pressures up to 12 atmospheric pressure.
7. Biocompatible material coated with preparatory thromboresistant material.
8. Gentle chronic tip facilitating insertion without traumatizing the tissue of the vessel.
9. Optional, ergonomically designed catheter tip to allow blood flow out or can be used as suction for aspiration of blood and/or other debris.

### Advantages.

The advantages of the double balloon retractable occlusion catheter are as follows:
1. **Short hospitalization time.** Most patients will be discharged within 24 to 48 hours, unlike the average 5 - 7 days for conventional procedures. Assuming a per day hospitalization costs $800 - $4,000 per day (average Canada and U.S. rates), this would amount to a savings of up to $10,000 - $20,000 per case.
2. **Less trauma.** The incision is made over the left chest, a small (mini) thoracotomy of 8 - 10 cm (rather than splitting the sternum), thus improving upon the comfort of the patient and associated risks.
3. **Faster recovery.** Patients experience a fast recovery as a result of the less invasive procedure, less trauma, shorter hospitalization, faster healing and decreased pain.
4. **Blood transfusion minimization and cost savings.** The blood transfusion requirements are reduced or eliminated. The total blood lost during the procedure is less than 400 cc.
5. **No CPB.** The complications of cardiopulmonary bypass vs. hemodilution, immune suppression, air emboli, atherosclerotic embolization, are avoided. (TM to obtain the cost of CPB). Performing bypass without CPB will allow those patients needing one graft (i.e., left anterior descending or right coronary) or patients with heavily calcified aortas.
6. Allows such re-operation in patients with previous operations, and patent (open) grafts.
7. No need for cross clamping. The risks of aortic cross clamping, the risks of which are significant in the elderly population, causing dissections and atherosclerotic embolization leading to neurologic events are completely avoided.
8. Patients with ethical and religious beliefs against transfusion.

Any features described or claimed herein may be used separately or in any combination except where clearly incompatible.

## Claims

1. A blood stopping catheter comprising:
a first elongate tubular member;
first and second inflatable balloons disposed in a spaced relation along the first elongate tubular member; and a second elongate tubular member communicating with said first and second inflatable balloons for inflation thereof.

2. A catheter as claimed in claim 1 wherein said first and second balloons are disposed at remote ends of the first elongate tubular member.

3. A catheter as claimed in claim 1 or claim 2 wherein the second elongate tubular member lies parallel to the first elongate tubular member.

4. A catheter as claimed in any one of claims 1 to 3 wherein the second elongate tubular member includes an inflation tube and a coupling tube in communication therewith.

5. A catheter as claimed in claim 4 wherein the coupling tube and the inflation tube have a T-shaped form.

6. A catheter as claimed in any one of claims 1 to 5 wherein the second elongate tube is smaller in diameter than the first elongate tube.

7. A catheter as claimed in any one of claims 4 to 6 wherein the coupling tube is foldable proximate to the first elongate tube for ease of insertion.

8. A use of a blood stopping catheter comprising
a first elongate tubular member;
first and second inflatable balloons disposed in a spaced relation along the first elongate tubular member; and
a second elongate tubular member communicating with said first and second inflatable balloons for inflation thereof so that when the catheter is inserted in an artery through an incision the first and second balloons are positioned on each side of the incision, and inflating the first and second balloons stops the blood flow from the incision while allowing blood flow through the first elongate tubular member.
